# EUROPÄISCHE PATENTSCHRIFT

(11) **EP 1 886 758 B1**
(45) Veröffentlichungstag und Bekanntmachung des Hinweises auf die Patenterteilung: **18.03.2009**
(21) Anmeldenummer: 06016465.4
(22) Anmeldetag: 07.08.2006
(51) Int. Cl.: B23K 26/04, A61F 9/01, A61F 9/007, A61B 5/00, A61F 9/008, A61B 17/00

(54) **Lasersystem für die refraktive Chirurgie**
Laser system for refractive surgery
Systeme laser pour chirurgie refractive

(43) Veröffentlichungstag der Anmeldung: 13.02.2008
(73) Patentinhaber: WaveLight AG, 91058 Erlangen (DE)
(72) Erfinder: Vogler, Klaus Dr., 90542 Eckental (DE)
(74) Vertreter: von Hellfeld, Axel

(56) Entgegenhaltungen:
- WO-A-20/04061425
- WO-A2-02/076355
- DE-A1- 10 020 559
- US-B1- 6 755 819
- LUBATSCHOWSKI H ET AL: "APPLICATION OF ULTRASHORT LASER PULSES FOR INTRASTROMAL REFRACTIVE SURGERY" GRAEFE'S ARCHIVE FOR CLINICAL AND EXPERIMENTAL OPHTHALMOLOGY, SPRINGER VERLAG, Bd. 238, Januar 2000 (2000-01), Seiten 33-39, XP001014554 ISSN: 0721-832X
- DREXLER W: "Ultrahigh-resolution optical coherence tomography" JOURNAL OF BIOMEDICAL OPTICS, Bd. 9, Nr. 1, Januar 2004 (2004-01), Seiten 47-74, XP002397794 ISSN: 1083-3668

## Beschreibung

Die Erfindung betrifft ein Lasersystem für die refraktive Chirurgie mit einer Laserstrahlquelle zum Erzeugen von Laserstrahlpulsen und optischen Mitteln zum Richten von Laserstrahlpulsen als Arbeitsstrahl auf oder in ein Auge.

In der Ophthalmologie wird unter "refraktiver Chirurgie" mit Lasern die Wechselwirkung von Laserstrahlung mit Teilen des Auges verstanden, um die brechenden Eigenschaften des Auges und damit seine Abbildungseigenschaften zur Beseitigung oder zumindest Linderung von Abbildungsfehlern zu ändern.

Ein besonders bedeutsames Beispiel der refraktiven Chirurgie ist die Korrektur der Fehlsichtigkeit eines Auges mit der LASIK-Technik. Bei der LASIK gemäß dem Stand der Technik wird zunächst die Hornhaut mittels eines Mikrokeratoms seitlich aufgeschnitten und das so entstehende Deckelchen (auch Flap genannt) wird zur Seite geklappt. Im so freigelegten Stroma der Hornhaut (Kornea) erfolgt mit Laserstrahlung eine sogenannte Ablation, d.h. Entfernung von Gewebe gemäß einem berechneten Ablationsprofil. Danach wird das Deckelchen zurückgeklappt und es erfolgt ein relativ schmerzfreier und schneller Heilungsprozess. Nach diesem Eingriff hat die Kornea andere Abbildungseigenschaften und die Fehlsichtigkeit ist behoben oder gemindert.

Üblicherweise erfolgt der oben beschriebene seitliche Einschnitt in die Kornea beim Stand der Technik mit einem sogenannten Mikrokeratom, d.h. einer oszillierenden mechanischen Schneide. In jüngerer Zeit wurden auch sogenannte Femtosekunden-Mikrokeratome eingesetzt, bei denen Femtosekunden-Laserpulse im Gewebe der Hornhaut fokussiert werden, um dort mit eng benachbarten Brennpunkten der Strahlung sogenannte laserinduzierte Fotodisruptionen im Hornhautgewebe zu erzeugen, die so durch das Hornhautgewebe geführt werden, dass letztlich ein Schnitt wie bei einem mechanischen Mikrokeratom entsteht.

Nachfolgend wird die vorliegende Erfindung mit Blick auf die vorstehend skizzierte LASIK-Technik näher erläutert. Ganz allgemein ist aber das erfindungsgemäße Lasersystem auch für andere Verfahren der refraktiven Chirurgie geeignet.

Die optische Kohärenztomographie (OCT) hat in jüngerer Zeit als diagnostisches Verfahren Eingang gefunden in die Ophthalmologie. So wurde die OCT eingesetzt für Messungen an der Retina *in vivo,* siehe Lynn M. Savage: "Adaptive optics improves OCT-based retinal imaging" in Biophotonics International, Dezember 2005, 48-49. Die mit dieser Technik an der Retina *in vivo* erfolgten Messungen liefern Bilder, die infolge einer axialen Schichtauflösung in der Tiefe Auskunft über Krankheitsbilder der Netzhaut liefern, bevor diese an der Netzhautoberfläche überhaupt erkennbar sind, beispielsweise mit der klassischen Spaltlampentechnik.

Eine weitere Anwendung der OCT in der refraktiven Chirurgie lehrt die US 6,755,819 B1. Dort wird die Fotoablation der Kornea durch ein OCT-Messverfahren begleitet, um die Stärke der Kornea während des Eingriffs zu messen und damit zu überwachen. Dort sind für den sogenannten Arbeitsstrahl, also den Laserstrahl, der die refraktive Chirurgie durchführt, einerseits und den Messstrahl für die OCT-Verfahren andererseits, unterschiedliche Strahlungsquellen vorgesehen.

Weitere Grundlagen und Anwendungen der OCT finden sich zum Beispiel in P. Hsiung, T.H. Ko, S. Bourquin, A. Aguirre, P. Herz und J. Fujimoto, "Optical Coherence Tomographie", in Biophotonics International, September 2003, 36-40.

Die DE 100 20 559 A1 offenbart einen Femtosekundenlaser dessen Strahl in einen selektiven Impulsverstärker eingespeist wird. Aus dem Verstärker können unverstärkte Impulse und verstärkte Impulse austreten. Die verstärkten Impulse und die unverstärkten Impulse können unterschiedliche Wiederholfrequenzen aufweisen. Die verstärkten Impulse werden einem zu bearbeitenden und zu untersuchenden Material zugeführt. Die unverstärkten Laserimpulse werden in einen Referenzarm eingestrahlt, in dem sie auf einen in definierter Weise hin und her beweglichen Referenzspiegel treffen. Vom Referenzspiegel und von der Oberfläche des zu untersuchenden Materials werden Teile der unverstärkten (Referenz)-Impulse zurückreflektiert und im Koppler überlagert. Nach Austritt aus dem Koppler werden die überlagerten unverstärkten Impulse einem Photodetektor zugeführt, in dem sich die Interferenz der überlagerten Impulse als periodische Variation des Detektorsignals detektieren lässt, wenn der Referenzspiegel mit im Wesentlichen konstanter Geschwindigkeit bewegt wird

Die Veröffentlichung "Ultrahigh-resolution optical coherence tomography" von Wolfgang Drexler, Journal of Biomedical Optics, Band 9, Nur.1, Januar 2004, Seite 47 bis 74, ISSN: 1083-3668 offenbart, dass die axiale Auflösung umgekehrt proportional zur spektralen Bandbreite der Lichtquelle sei.

Der Erfindung liegt die Aufgabe zugrunde, ein Lasersystem für die refraktive Chirurgie bereitzustellen, das mit vergleichsweise einfachen Mitteln eine optische Kohärenztomographie vor, während oder nach dem refraktiven Eingriff ermöglicht.

Die Aufgabe wird durch ein Lasersystem gemäß Anspruch 1 gelöst.

Hierzu stellt die Erfindung ein Lasersystem bereit mit einer Strahlquelle zum Erzeugen von Laserstrahlpulsen und optischen Mitteln zum Richten der Laserstrahlpulse auf oder in ein Auge, wobei mittels zumindest eines der optischen Mittel ein Teil der erzeugten Laserstrahlpulse als Messstrahl in eine Einrichtung zur optischen Kohärenztomographie abgezweigt wird.

Gemäß der Erfindung kann also eine zusätzliche OCT-Messstrahlquelle, neben der Arbeitsstrahlquelle, entfallen.

Eine bevorzugte Ausgestaltung der Erfindung sieht Mittel vor zum Erhöhen der spektralen Bandbreite der für die Messung abgezweigten Laserstrahlpulse. Wird als gemeinsame Quelle für den Arbeitsstrahl und den Messstrahl ein Femtosekundenlaser eingesetzt, dann kann dieser Laser zum Beispiel für den oben beschriebenen LASIK-Schnitt in der Kornea verwendet werden und über einen Strahlteiler wird der Messstrahl für die optische Kohärenztomographie abgezweigt. Für die OCT ist ein spektral möglichst breitbandiger Messstrahl förderlich. Die spektrale Bandbreite des Messstrahls bestimmt das axiale Auflösungsvermögen der OCT gemäß dem Wiener-Tschinchin-Theorem (R. Böhm und E. Overbeck "Das Spektrum eines LED und die Leistung einer Laserdiode, OCT-Untersuchung von dünnen Schichten" in Laser & Photonic 5/2005, 28-31.). Deshalb wird mit der erfindungsgemäßen spektralen Erhöhung der Bandbreite der abgezweigten Messstrahlung ein Femtosekunden-Kontinuum erzeugt welches es erlaubt, eine spektral sehr breitbandige Emission zu generieren, zum Beispiel in einer Single-Mode-Fibre. Mit Bandbreiten größer als 400 nm und insbesondere größer als 800 nm werden axiale Tiefenauflösungen von besser als 1 µm erreicht. Das axiale Tiefenauflösungsvermögen beschreibt die Auflösung der Messung in Richtung des Laserstrahls, also in der Tiefe des untersuchten Gewebes.

Eine weitere Ausgestaltung der Erfindung sieht Mittel vor zum Darstellen von mit der optischen Kohärenztomographie gewonnenen Daten, zum Beispiel Bildern, während der refraktiven Chirurgie, sodass der Arzt während des Prozesses Auskunft erhält über das jeweils vorliegende operative Ergebnis.

Besonders geeignet ist die Erfindung zum Schneiden des oben erläuterten Flaps bei der LASIK. Werden die vorstehend beschriebenen hohen axialen Auflösungen im Bereich von 1 µm erreicht, dann kann damit auch die Qualität des LASIK-Schnittes überwacht werden und insbesondere können auch Rauhigkeiten aufgrund der Fotodisruptionen überwacht werden, d.h. es kann die Glätte des Schnittes überwacht werden.

Mit diesen Maßnahmen erreicht die Erfindung ohne eine gesondere (und teure) OCT-Messstrahlquelle ein hohes Auflösungsvermögen bei der Messung.

Eine Variante der Erfindung sieht einen wahlweise betätigbaren Verschluss im Strahlengang des Arbeitsstrahles vor, um wahlweise eine OCT unabhängig von einer Laserbehandlung durchführen zu können, z.B. vor oder nach dem LASIK-Schnitt.

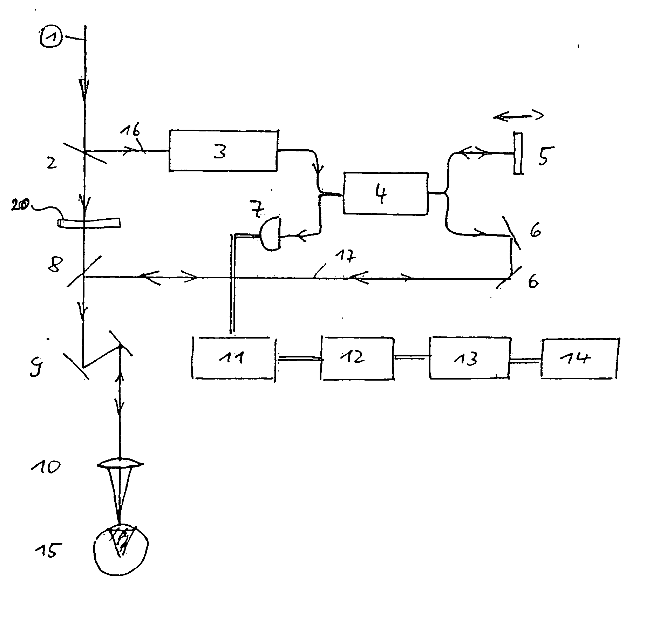

Nachfolgend wird ein Ausführungsbeispiel der Erfindung anhand der Zeichnung näher erläutert.

Die einzige Figur zeigt schematisch ein Lasersystem für die refraktive Chirurgie mit integrierter optischer Kohärenztomographie (OCT).

Das Ausführungsbeispiel betrifft ein Femtosekunden-Lasersystem als Mikrokeratom, um in Echtzeit ("on-line") während eines Flap-Schnitts Messungen in der Kornea vorzunehmen, also insbesondere Messungen über den geometrischen Verlauf des Schnitts und/oder die Rauhigkeit des Schnitts. Auch die Abmessungen des Flaps und der verbleibenden Hornhaut können gemessen werden (Pachymetrie).

Eine Laserstrahlquelle liefert in an sich bekannter Weise Laserstrahlpulse 1 mit einer Wellenlänge von 1035 nm (λ₁), einer Pulslänge von 400 fs, 5 bis 10 µJ Energie, und einer Wiederholrate von 200 kHz. Diese Laserstrahlpulse 1 werden in an sich bekannter Weise zur oben beschriebenen Erzeugung eines LASIK-Flaps eingesetzt. Diese Laserstrahlpulse heißen hier Arbeitsstrahl. Der Arbeitsstrahl wird in an sich bekannter Weise über einen Scanner 9 und eine Fokussierlinse auf das Auge 15 des Patienten gerichtet. Die fokussierte Strahlung erzeugt im Gewebe der Kornea die beschriebenen Fotodisruptionen und durch enges Nebeneinandersetzen der einzelnen Brennpunkte der Pulse erfolgt der Schnitt im Gewebe. Die Brennpunkte werden also in einer x-y-Ebene so gesetzt, dass ein aufklappbares Flap erzeugt wird.

Erfindungsgemäß werden aus den Laserpulsen 1 Messpulse 16 mit einem Strahlteiler 2 abgezweigt. Der Strahlteiler 2 koppelt 5 bis 10% der einfallenden Strahlung in den Messstrahl 16 aus.

Im Messstrahl 16 sind Mittel 3 angeordnet, um ein Femtosekunden-Kontinuum zu generieren mit der Wellenlänge λ₂. Wie oben erläutert ist, wächst das Auflösungsvermögen einer OCT-Messung mit der spektralen Bandbreite des Messstrahls. Die Mittel 3 erzeugen also ein Femtosekunden-Kontinuum mit einer sehr breitbandigen Emission mit Bandbreiten von mehr als 400 nm. Mit Single-Mode-Fibern lassen sich zur Zeit Bandbreiten von mehr als 400 nm bis hin zu 800 nm erzielen (vgl. J. Posthumus, "Modelocked Fibre Lasers Aid Short Pulse Creation", in Optics. Org, OLE Product Guide 2006). Single-Mode-Fibres sind auch beschrieben im Datenblatt der Firma FEMTOLASERS ProduktionsGmbH, Integral OCT.

Die Mittel 3 erzeugen somit breitbandige Strahlung um eine Wellenlänge von 1035 nm, einer Pulslänge von 400 fs, und mit einer Pulsenergie von 1 µJ sowie einer Bandbreite (FWHM) von 400 bis 800 nm. Dies liefert ein axiales Tiefenauflösungsvermögen der nachfolgend beschriebenen OCT von besser als 1 µm.

Die in der Figur mit 3, 4, 5, 6 und 7 bezeichneten Bauelemente bilden eine als solches bekannte OCT-Messanordnung aus einem faseroptischen Strahlteiler 4, einem beweglichen Spiegel 5, Umlenkspiegeln 6 für den Messarm und einem Fotodetektor 7. Die Anordnung entspricht einem Michelson-Interferrometer. Der Spiegel 5 ist beweglich und je nach der optischen Weglänge, auf die der Referenzspiegel 5 eingestellt ist, misst der Detektor 7 konstruktive oder destruktive Interferenzen.

Über die Umlenkspiegel wird der breitbandige OCT-Messstrahl 17 auf einen dichroitischen Spiegel 8 gelenkt, der den OCT-Messstrahl 17 mit dem Arbeitsstrahl räumlich genau überlagert. Somit wird auch der OCT-Messstrahl 17 genau wie der Arbeitsstrahl mit dem Scanner 9 und der Fokussierlinse 10 in das Auge 15 des Patienten gerichtet. In den Tiefenschichten der Probe (hier des Auges 15) reflektierte OCT-Messstrahlung gelangt über den dichroitischen Spiegel 8 zurück in die OCT-Messanordnung und, je nach Stellung des Spiegels 5, werden die genannten konstruktiven oder destruktiven Interferenzen am Detektor 7 gemessen. Das Ausgangssignal des Fotodetektors 7 gelangt über einen Verstärker 11 in einen Bandpassfilter 12 und dann in einen Rechner 13 zur Durchführung einer für die OCT-Messung an sich bekannten schnellen Fourier-Analyse. Die so erzeugte Darstellung auf einem Monitor 14 liefert nicht nur ein Tiefenprofil der Kornea, in dem die vom Femtosekunden-Arbeitsstrahl erzeugte Schnittebene erkennbar ist, sondern infolge der Auslenkung (des Scannens) zusammen mit dem Arbeitsstrahl mittels des Scanners 9 auch ein in Querrichtung (z-Richtung) lateral aufgelöstes und somit ein dreidimensionales Tiefenprofil bei ausreichender räumliche Kohärenz und Wellenfrontqualität.

Der abgezweigte Messstrahl 16 kann eine höhere Repetitionsrate und deutlich geringere Pulsenergie, nämlich sehr viel weniger als 1 µJ, als der refraktiv wirkende energiereichere Arbeitsstrahl aufweisen, zum Beispiel die Puls-Wiederhol-Rate des Fs-Oszillators (eine Pulswiederholrate von 1 MHz oder mehr), während der refraktiv wirkende Arbeitsstrahl eine Pulswiederhohlrate von 200 kHz bei einer Pulsenergie von mehr als 5 µJ aufweist. Damit ist die Beeinträchtigung des refraktiv wirkenden Arbeitsstrahls durch den Messstrahl vernachlässigbar. Mit den Parametern des Messstrahls ist eine sogenannte Selbstphasenmodulation in einer Single-Mode-Faser mit einem Durchmesser von 5 bis 10 µm durchaus möglich. Wird für den Messstrahl 16 eine höhere Repetitionsrate als für den refraktiven Arbeitsstrahl vorgesehen, dann erfolgt die Auskopplung des Messstrahles vorzugsweise im Femtosekunden-Lasersystem selbst, dort zum Beispiel zwischen dem Oszillator und dem Verstärker.

Der abgezweigte Messstrahl kann auch unabhängig vom Arbeitsstrahl genutzt werden, zum Beispiel vor oder nach dem LASIK-Schnitt, indem der Arbeitsstrahl durch einen Schutter (oder eine Strahlsenke "beam dump") 20 geblockt wird.

Das dargestellte Messverfahren ermöglicht es, Rauhigkeiten des Flap-Schnitts bei LASIK, die durchaus im Bereich von mehreren µm liegen können, zu erkennen. Auch durch Fotodisruption erzeugte Gasblasen sollten erkennbar sein. Die geometrischen Verhältnisse der Randflächen der Kornea als auch in der Kornea lassen sich auflösen, also insbesondere die Schnitttiefe und auch der Randschnitt ist zu vermessen. Neben der Rauhigkeit des Flap-Bettes, kann auch der Randschnitt, zum Beispiel der Neigungswinkel, erkannt werden.

## Patentansprüche

1. Lasersystem für die refraktive Chirurgie mit einer Laserstrahlquelle (1) zum Erzeugen von Laserstrahlpulsen und mit optischen Mitteln (2, 8, 9, 10), um Laserstrahlpulse als Arbeitsstrahl auf oder in ein Auge (15) zu richten, wobei mittels zumindest eines der optischen Mittel (2) ein Teil der erzeugten Laserstrahlpulse als Messstrahl (16) in eine Einrichtung (3, 4, 5, 7) zur optischen Kohärenztomographie abgezweigt wird,
**gekennzeichnet durch** Mittel (3) zum Erhöhen der spektralen Bandbreite der abgezweigten Laserstrahlpulse des Messstrahls (16).

2. Lasersystem nach Anspruch 1, **dadurch gekennzeichnet, dass** die optischen Mittel (9, 10) für einen LASIK-Schnitt eingerichtet sind.

3. Lasersystem nach einem der vorhergehenden Ansprüche, **dadurch gekennzeichnet, dass** die Laserstrahlquelle Femtosekundenlaserpulse emittiert.

4. Lasersystem nach einem der vorhergehenden Ansprüche, **dadurch gekennzeichnet, dass** die optischen Mittel (9, 10) für eine Korneaablation eingerichtet sind.

5. Lasersystem nach Anspruch 1 mit Mitteln (11, 12, 13, 14) zum Darstellen von mit der optischen Kohärenztomographie gewonnenen Daten während der refraktiven Chirurgie.

6. Lasersystem nach einem der vorhergehenden Ansprüche mit einem wahlweise betätigbaren Verschluss (20) im Strahlengang des Arbeitsstrahls.

## Claims

1. A laser system for refractive surgery having a laser beam source (1) for generating laser beam pulses and having optical means (2, 8, 9, 10) to direct laser beam pulses as a working beam on or into an eye (15), wherein by at least one of the optical means (2) a portion of the generated laser beam pulses is diverted as a monitoring beam (16) into a means (3, 4, 5, 7) for optical coherence tomography,
**characterized by** means (3) for increasing the spectral band width of the diverted laser beam pulses of the monitoring beam (16).

2. The laser system according to claim 1, **characterized in that** the optical means (9, 10) are configured for a LASIK incision.

3. The laser system according to any one of the preceding claims, **characterized in that** the laser beam source emits femtosecond laser pulses.

4. The laser system according to any one of the preceding claims, **characterized in that** the optical means (9, 10) are configured for ablation of the cornea.

5. The laser system according to claim 1 including means (11, 12, 13, 14) for displaying data attained with the optical coherence tomography during refractive surgery.

6. The laser system according to any one of the preceding claims with a selective operatable shutter (20) in the optical path of the working beam.

## Revendications

1. Système laser pour la chirurgie réfractive, comprenant une source de rayon laser (1) permettant de générer des impulsions de rayon laser, ainsi que des moyens optiques (2, 8, 9, 10) permettant de diriger lesdites impulsions de rayon laser en tant que rayon de travail sur ou dans un oeil (15), une partie des impulsions de rayon laser générées étant dérivée en tant que rayon de mesure (16) vers un dispositif (3, 4, 5, 7) par au moins un des moyens optiques (2), pour une tomographie optique cohérente,
**caractérisé par** des moyens (3) permettant d'augmenter la largeur de bande spectrale des impulsions laser du rayon de mesure dérivé (16).

2. Système laser selon la revendication 1, **caractérisé en ce que** les moyens optiques (9, 10) sont conçus pour une intervention LASIK.

3. Système laser selon l'une des revendications précédentes,
**caractérisé en ce que** la source de rayon laser émet des impulsions laser femto-seconde.

4. Système laser selon l'une des revendications précédentes, **caractérisé en ce que** les moyens optiques (9, 10) sont conçus pour une ablation de la cornée.

5. Système laser selon la revendication 1, muni de moyens (11, 12, 13, 14) permettant la représentation de données acquises par tomographie optique cohérente pendant l'intervention chirurgicale réfractive.

6. Système laser selon l'une des revendications précédentes, muni d'un obturateur (20) pouvant être sélectivement actionné dans le trajet du rayon de travail.
